# EUROPEAN PATENT APPLICATION

(11) **EP 4 299 003 A1**
(43) Date of publication of application: **03.01.2024**
(21) Application number: 22182282.8
(22) Date of filing: 30.06.2022
(51) Int. Cl.: A61B 5/08, A61B 5/1455, A61B 5/00

(54) **SYSTEM AND METHOD FOR CONTROLLING A PULSE OXIMETRY SENSOR**

(71) Applicant: Koninklijke Philips N.V., 5656 AG Eindhoven (NL)
(72) Inventor: DOS SANTOS DA FONSECA, Pedro Miguel Ferreira, Eindhoven (NL); VAN DEN ENDE, Daan Anton, Eindhoven (NL); VAN ZANTEN, Joyce, 5656AG Eindhoven (NL)
(74) Representative: Philips Intellectual Property & Standards

(57) **Abstract**

A system and method for controlling a pulse oximetry sensor. The pulse oximetry sensor is activated and de-activated according to one or more breathing characteristics of a subject's breathing during sleep such that the pulse oximetry sensor acquires pulse oximetry data only at times for which the one or more breathing characteristics indicate that oxygen desaturation is likely to occur.

## Description

### FIELD OF THE INVENTION

The invention relates to the field of pulse oximetry, and, in particular, to controlling pulse oximetry sensors.

### BACKGROUND OF THE INVENTION

There is an increasing prevalence of, and interest in, obstructive sleep-disordered breathing (SDB). This is a syndrome of upper airway dysfunction during sleep that is characterized by snoring and/or increased respiratory effort secondary to greater upper airway resistance and pharyngeal collapsibility.

A subset of SDB patients suffer from obstructive sleep apnea (OSA) for which treatment is required. Traditionally, OSA diagnosis is performed by conducting a PSG in a sleep lab or a home sleep apnea test (HSAT), which involves measuring multiple parameters, including respiratory airflow, chest movement and heart rate, involving a dedicated measurement device. Such tests are typically prescribed by sleep physicians based on the symptoms of patients requesting a consult.

However, this approach is cumbersome, time-consuming and resource intensive. Moreover, many people do not seek medical care for their disorder, because they are unaware and do not take action themselves.

A higher coverage can be achieved by applying focused screening techniques that indicate to a person that they may be suffering from OSA using measurements from medical or consumer grade wearables.

To this end, wearables that measure sleep disordered breathing have been developed. A common method for screening for OSA and similar conditions is to monitor oxygen desaturation events during sleep and provide feedback to the user to guide them towards a proper diagnosis if needed.

A conventional approach for measuring oxygen desaturation events or levels is through the use of pulse oximetry. Traditionally, a pulse oximetry sensor is able to estimate the amount of oxygen in the blood by optically probing the peripheral capillary bed, e.g., using transmissive or reflective photoplethysmography (PPG). The principles for measuring pulse oximetry using such techniques are well known in the art.

There is an ongoing desire to improve the efficiency of pulse oximetry sensor and the clinical relevance of pulse oximetry data.

### SUMMARY OF THE INVENTION

The invention is defined by the claims.

According to examples in accordance with an aspect of the invention, there is provided a processing system for controlling a pulse oximetry sensor, the processing system being configured to: obtain, from a detector different to the pulse oximetry sensor, breathing data responsive to a subject's breathing during a sleep session of the subject; process the breathing data to determine one or more breathing characteristics; and control the pulse oximetry sensor responsive to the one or more breathing characteristics.

The inventors have recognized that a subject's breathing pattern during sleep, in particular whether and how the subject is snoring, may be indicative of sleep disordered breathing. By controlling the pulse oximetry sensor according to the subject's breathing pattern, the pulse oximetry sensor may be turned on (i.e. acquire pulse oximetry data) only when the subject's breathing pattern indicates obstructive sleep-disordered breathing.

This method of controlling a pulse oximetry sensor reduces a time for which the pulse oximetry sensor is turned on/active during a sleep session, thus reducing power consumption, while still reliably detecting oxygen desaturation events. The reduced power consumption allows a pulse oximetry sensor to acquire pulse oximetry data over a longer period (e.g. an entire sleep session) with a smaller battery.

The proposed approach also means and/or ensures that the recorded pulse oximetry data is relevant for assessing OSA, as it will directly relate to snoring events and other breathing patterns that are characteristic of sleep disordered breathing. This can, for instance, make post-processing or analysis of the pulse oximetry data acquired or sampled by the pulse oximetry sensor more efficient.

In some examples, the detector comprises at least one of: a microphone, a resonator, an accelerometer, a gyroscope, an inertial measurement unit, a pressure sensor, a flow sensor, a seismocardiographic sensor, and/or a ballistocardiographic sensor.

For example, breathing data can be obtained from one or more contact microphones (i.e. in contact with the subject, e.g. on the subject's neck or chest) or non-contact microphones (positioned in the vicinity of the subject, e.g. a microphone in the subject's smartphone, placed on a nightstand near the subject). A resonator (such as the resonators used in stethoscopes) may also be used to obtain breathing data (e.g. a resonator comprising a diaphragm that makes contact with a transducer, such as a piezoelectric crystal, when the diaphragm vibrates, or a resonator comprising an electromagnetic diaphragm and conductive plate that form a capacitive sensor). Other sensors that detect movements/vibrations associated with breathing may also be used to detect breathing data (e.g. one or more accelerometers, gyroscopes, inertial measurement units (IMUs), pressure sensors, flow sensors, seismocardiographic sensors and/or ballistocardiographic sensors).

In some examples, the processing system is configured to control the pulse oximetry sensor by: processing the one or more breathing characteristics to determine whether the subject is snoring; and controlling the pulse oximetry sensor to start acquiring pulse oximetry data in response to a determination that the subject is snoring.

This provides a simple mechanism to control the pulse oximetry sensor based on the subject's snoring. The inventors have recognized that snoring often occurs during a hypopnea, immediately before an obstructive apnea event, and/or shortly after an obstruction has been resolved. By controlling the pulse oximetry sensor to start acquiring pulse oximetry data (i.e. turning on/activating the pulse oximetry sensor) as soon as it is determined that the subject has started snoring, pulse oximetry data will be acquired at times when it is likely that oxygen desaturation is occurring. Not acquiring pulse oximetry data before the subject has started snoring reduces the time that the pulse oximetry sensor is switched on/active, thus reducing power consumption of the pulse oximetry sensor, and, since it is unlikely that oxygen desaturation will occur before the subject has started snoring, the pulse oximetry sensor is unlikely to miss detection of oxygen desaturation events using this control mechanism. In this way, the power consumption of the pulse oximetry sensor over a sleep session is reduced while maintaining an accuracy of the pulse oximetry data.

In some examples, the processing system is configured to control the pulse oximetry sensor by: processing the one or more breathing characteristics to determine whether the subject is snoring; and, in response to a determination that the subject is snoring: processing the one or more breathing characteristics to identify a type of snoring; and controlling the pulse oximetry sensor according to the identified type of snoring.

The inventors have recognized that a subject's snoring has different characteristics during regular breathing, during hypopnea events, and before and after obstructive apnea events. By identifying which of these is indicated by the subject's current snoring, the processing system can determine a most suitable time (i.e. a time at which oxygen desaturation is likely to occur) to acquire pulse oximetry data with even greater precision, further reducing the power consumption of the pulse oximetry sensor without reducing an accuracy of the data obtained.

In some examples, the processing system is configured to, in response to the identified type of snoring being a first type of snoring that occurs during hypopnea: control the pulse oximetry sensor to start acquiring pulse oximetry data.

Snoring typically continues during a hypopnea event; this snoring will have different characteristics to snoring that occurs before and after an apnea event. By identifying that a hypopnea event is occurring based on characteristics of the subject's breathing and controlling the pulse oximetry sensor to acquire pulse oximetry data during the hypopnea event, pulse oximetry data is obtained at a time when an oxygen desaturation event is likely.

In some examples, the processing system is configured to, in response to the identified type of snoring being a second type of snoring that occurs before an obstructive apnea event: process the one or more breathing characteristics to determine a time since a most recent snore; and control the pulse oximetry sensor to start acquiring pulse oximetry data in response to a determination that the time since a most recent snore exceeds a first predetermined time period.

In other words, when the subject's snoring indicates that an obstructive apnea event is about to occur, the pulse oximetry sensor may be controlled to start acquiring pulse oximetry data once the subject has stopped snoring. An obstructive apnea is a cessation of breathing, so a subject will not snore during an obstructive apnea. The cessation of snoring may therefore indicate the start of an obstructive apnea event, and is therefore a suitable time to start acquiring pulse oximetry data.

The first predetermined time period, used to determine a cessation of snoring caused by an obstructive apnea event, may, for example, be less than the length of one breathing cycle, e.g. the length of half of one breathing cycle. For instance, assuming the subject has a breathing rate of 12 breaths per minute, the first predetermined period may be 2.5 seconds. In some examples, the processing system may process the breathing data for the subject to determine the subject's breathing rate, and determine a subject-specific first predetermined time period based on the determined breathing rate. In other examples, the first predetermined time period may be determined based on typical breathing rate (e.g. a population average).

In some examples, the processing system is configured to, in response to the identified type of snoring being a third type of snoring that occurs after an obstruction is resolved: control the pulse oximetry sensor to start acquiring pulse oximetry data.

After an obstruction has been resolved, the subject's oxygen saturation level will take a while to return to a normal level. By starting acquiring pulse oximetry data as soon as it is determined that the subject's snoring indicates that an obstruction has been resolved, the pulse oximetry data may be obtained before oxygen saturation has returned to normal level.

In some examples, the processing system is configured to identity a type of snoring based on subject-specific data obtained during at least one previous sleep session for the subject.

The breathing characteristics indicative of each type of snoring may vary between subjects. By learning the breathing characteristics for each type of snoring for the subject, the accuracy of the identification of the type of snoring may be improved.

For instance, during a diagnostic sleep session for the subject, breathing data may be obtained throughout the session, and obstructive apnea and hypopnea events that occur during the session may be detected (e.g. using PSG in a sleep lab or using a home sleep apnea test). The breathing data and detected obstructive apnea and hypopnea events may then be processed to determine one or more breathing characteristics (e.g. spectral properties) typical of the subject's breathing during hypopnea events, and before and after apnea events.

In some examples, the processing system is configured to identify a type of snoring using a machine-learning algorithm, wherein the machine-learning algorithm has been trained using the subject-specific data obtained during at least one previous sleep session for the subject.

For example, a pulse oximetry sensor may acquire pulse oximetry data continuously at the start of the machine-learning algorithm's training, and active reinforcement learning may be used to maximize the detection of oxygen desaturation events while minimizing the time for which the pulse oximetry sensor is active. In other words, the machine-learning algorithm may be trained to identify types of snoring associated with regular breathing, hypopnea events, and before and after obstructive apnea events by rewarding the machine-learning algorithm for activating the pulse oximetry sensor only when oxygen desaturation occurs, and punishing the machine-learning algorithm when the pulse oximetry sensor acquires pulse oximetry data for a period during which the subject's oxygen saturation level remains high.

In some examples, the processing system is configured to control the pulse oximetry sensor by: processing the one or more breathing characteristics to determine whether the subject is snoring; and, in response to a determination that the subject is snoring: processing the one or more breathing characteristics to determine a running variance for one or more snore features; in response to a determination that each running variance exceeds a predetermined running variance threshold, controlling the pulse oximetry sensor to acquire pulse oximetry data; and in response to a determination that the running variance for one or more snore features does not exceed the predetermined threshold, controlling the pulse oximetry sensor not to acquire pulse oximetry data.

In other words, the pulse oximetry sensor may be switched on and off (or activated/deactivated) according to whether the running variance does or does not exceed a threshold.

It has been found that irregular snoring, and therefore an increased running variance for the time interval between consecutive snores, is characteristic of apneic snoring. The use of a running variance threshold to control the pulse oximetry sensor therefore provides a simple mechanism for acquiring pulse oximetry data during periods of apneic snoring while not acquiring pulse oximetry data during periods of regular snoring.

This method of controlling the pulse oximetry sensor may be particularly effective for heavy snorers with long apneic phase durations.

In some examples, the processing system is configured to control the pulse oximetry sensor by: processing the one or more breathing characteristics to determine a time since a most recent snore; and controlling the pulse oximetry sensor to stop acquiring pulse oximetry data in response to a determination that the time since a most recent snore exceeds a second predetermined time period.

In other words, the pulse oximetry sensor may stop acquiring pulse oximetry data (i.e. be turned off/deactivated) once an interval has elapsed from the end of a period of snoring. Once this interval has passed, oxygen desaturation events are unlikely to occur, so the pulse oximetry data can be turned off/deactivated in order to conserve power without missing the detection of oxygen desaturation events.

The second predetermined time period should be long enough, not only to determine that the subject has stopped snoring, but to allow pulse oximetry data to be acquired in the period immediately after the subject has stopped snoring, as this period may coincide with an obstructive apnea event. For instance, the second predetermined time period may have a length of 15-30 seconds (e.g. a length of 20-25 seconds).

In some examples, the processing system is configured to control the pulse oximetry sensor by: obtaining pulse oximetry data from the pulse oximetry sensor; processing the pulse oximetry data to determine a measure of oxygen saturation; and controlling the pulse oximetry sensor to stop acquiring pulse oximetry data in response to a determination that the measure of oxygen saturation exceeds an oxygen saturation threshold throughout a third predetermined time period.

In other words, the pulse oximetry sensor may be turned off/deactivated if no oxygen desaturation is detected within a period in which oxygen desaturation would be expected had an obstructive apnea or hypopnea event occurred.

Oxygen desaturation is detected if the measure of oxygen saturation falls below the oxygen saturation threshold. The oxygen saturation threshold may be a predetermined threshold (e.g. 95%), or may be a subject-specific threshold determined based on a value of the measure of oxygen saturation for the subject measured during regular breathing.

The length of the third predetermined time period may depend on the mechanism used to turn on/activate the pulse oximetry sensor. For example, the third predetermined time period may be longer where the pulse oximetry sensor acquires pulse oximetry data whenever the subject is snoring, as the snoring may precede an obstructive apnea event, in which case the oxygen saturation level would not fall below the oxygen saturation threshold until a few seconds after snoring has ceased. In cases where the pulse oximetry sensor is controlled to start acquiring pulse oximetry data according to an identified type of snoring, the pulse oximetry sensor is more likely to start acquiring the pulse oximetry data once an obstructive apnea or hypopnea event has started, and so the third predetermined time period may be shorter (for example, less than 15 seconds, e.g. 10 seconds).

There is also proposed a system for measuring oxygen saturation, comprising: a pulse oximetry sensor; a detector capable of obtaining breathing data responsive to a subject's breathing during a sleep session of the subject; and the processing system described above.

According to another aspect of the invention, there is provided a computer-implemented method for controlling a pulse oximetry sensor, the computer-implemented method comprising: obtaining, from a detector different to the pulse oximetry sensor, breathing data responsive to a subject's breathing during a sleep session of the subject; processing the breathing data to determine one or more breathing characteristics; and controlling the pulse oximetry sensor responsive to the one or more breathing characteristics.

There is also proposed a computer program product comprising computer program code means which, when executed on a computer device having a processing system, cause the processing system to perform all of the steps of the method described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates a system for measuring oxygen saturation, according to an embodiment of the invention; and
Figure 2 illustrates a computer-implemented method for controlling a pulse oximetry sensor, according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

There is proposed a system and method for controlling a pulse oximetry sensor. The pulse oximetry sensor is activated and de-activated according to one or more breathing characteristics of a subject's breathing during sleep such that the pulse oximetry sensor acquires pulse oximetry data only at times for which the one or more breathing characteristics indicate that oxygen desaturation is likely to occur.

Embodiments are at least partly based on the realization that breathing characteristics, in particularly breathing characteristics relating to snoring, are indicative of sleep disordered breathing, and that controlling a pulse oximetry sensor to acquire pulse oximetry data only during periods of sleep disordered breathing reduces power consumption of the pulse oximetry sensor over a sleep session without reducing the accuracy or reliability of the data obtained by missing oxygen desaturation events.

Illustrative embodiments may, for example, be employed in systems for measuring oxygen saturation using a pulse oximetry sensor, and in particular in systems that use a wearable, wireless pulse oximetry sensor that is powered by a battery.

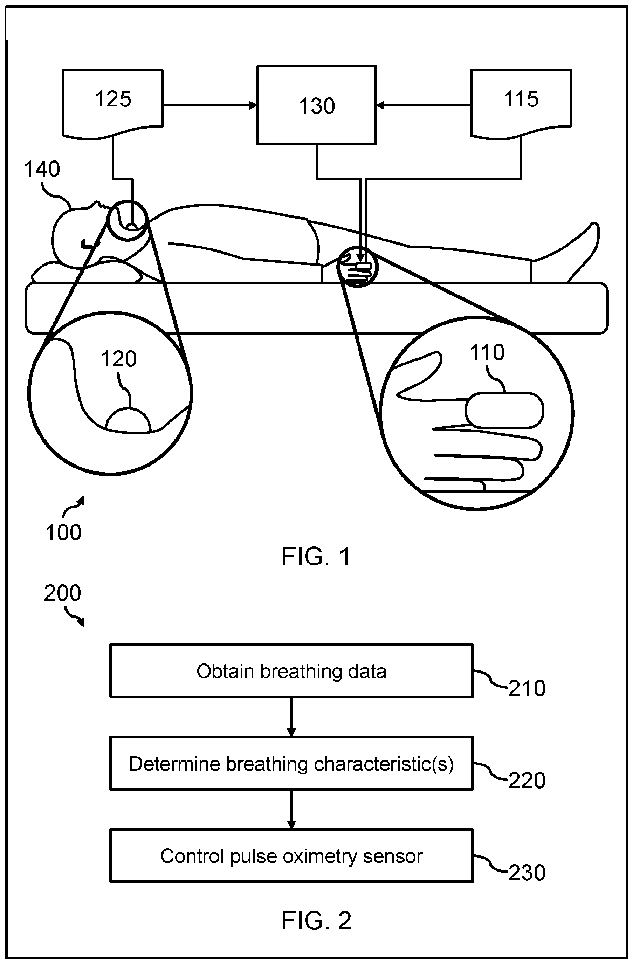

Figure 1 illustrates a system 100 for measuring oxygen saturation, according to an embodiment of the invention. The system 100 comprises a pulse oximetry sensor 110, a (different) detector 120 capable of obtaining breathing data 125, and a processing system 130 for controlling the pulse oximetry sensor. The processing system may be located in a same device as the pulse oximetry sensor and/or the detector, or in a separate device. The processing system is, itself, an embodiment of the invention.

In Figure 1, the pulse oximetry sensor 110 is a wireless pulse oximetry sensor worn on a subject's fingertip; however, the pulse oximetry sensor may be any sensor capable of acquiring pulse oximetry data for a subject during a sleep session of the subject. For example, the pulse oximetry sensor may be incorporated into or mounted on a ring worn on a subject's finger or thumb, a band or strap worn around a subject's wrist, or a clip worn on a subject's ear. The invention is particularly suited to batterypowered pulse oximetry sensors, as such sensors benefit from the reduced power consumption provided by the invention.

The detector 120 obtains breathing data 125 for a subject 140. The breathing data is responsive to the subject's breathing during a sleep session of the subject. In Figure 1, the detector is a microphone placed on the subject's neck that detects the sound of the subject's breathing; however, the detector may be any kind of detector capable of obtaining data responsive to a subject's breathing. For example, the detector may comprise at least one of: a (contact and/or non-contact) microphone, a resonator, an accelerometer, a gyroscope, an inertial measurement unit (IMU) (e.g. a MEMS IMU), a pressure sensor, a flow sensor, a seismocardiographic sensor, and/or a ballistographic sensor. Further examples of suitable detectors for obtaining breathing data will be apparent to the skilled person.

Contact microphones placed on/against the subject's body (e.g. on the subject's neck or chest) and/or non-contact microphones in the vicinity of the subject (e.g. a microphone in a smartphone kept close to the subject's bed) may be used to detect breathing sounds. In some examples, a microphone may be contained in the same device as the pulse oximetry sensor.

Similarly, one or more resonators (e.g. of a type suitable for use in a stethoscope) placed on/against the subject's body may be used to detect breathing sounds. Suitable resonators include resonators having a diaphragm that connects to a transducer (e.g. a piezoelectric crystal) when vibrating, and/or resonators having a diaphragm made of an electromagnetic material that forms a capacitive sensor with a conductive plate.

Other detectors, such as accelerometers, gyroscopes, IMUs, pressure sensors, flow sensors, and/or seismocardiographic sensors placed on or against the subject's body, and/or bed-based ballistocardiographic sensors, may be used to detect vibrations and/or other movements associated with breathing. The use of such detectors to obtain data responsive to a subject's breathing is well known. Further suitable detectors for obtaining data responsive to a subject's breathing will be apparent to the skilled person.

The processing system 130 obtains the breathing data 125 from the detector 120, and processes the breathing data to determine one or more breathing characteristics. The one or more breathing characteristics may comprise characteristics that may be used to determine whether or not a subject is snoring, such as a power in a predefined frequency band (e.g. a high frequency band) corresponding to typical snoring, an overall power, and/or an amplitude of the signal envelope over a predefined time period (e.g. a few seconds). The one or more breathing characteristics may additionally comprise characteristics that may be used to identify a type of snoring, such as a fundamental frequency of the signal carrying the data, an amplitude of the signal envelope, a power of the signal, and/or changes in frequency, amplitude and/or power over the duration of an identified snore and/or between consecutive snores.

The processing system 130 then controls the pulse oximetry sensor 110 responsive to the one or more breathing characteristics. The processing system controls the pulse oximetry sensor 110 to acquire pulse oximetry data 115 only at times when the one or more breathing characteristics indicate that an obstructive apnea or hypopnea event is likely to occur.

In some examples, the processing system 130 may control the pulse oximetry sensor 110 by processing the one or more breathing characteristics to determine whether or not the subject 140 is breathing. For instance, the processing system may control the pulse oximetry sensor to acquire pulse oximetry data 115 in response to a determination that the subject has stopped breathing for a time period exceeding a predetermined breathing cessation period (e.g. the pulse oximetry data may be controlled to start acquiring pulse oximetry data in response to a determination that no breathing has been detected in the breathing data for a period of at least 10 seconds). A period (e.g. of at least 10 seconds) during which no breathing occurs is characteristic of an obstructive apnea event, during which there is no airflow.

In some examples, the processing system 130 may control the pulse oximetry sensor 110 by processing the one or more breathing characteristics to determine whether the subject 140 is snoring.

Methods for detecting snoring based on data responsive to a subject's breathing are well known, and therefore methods for performing the steps of determining one or more suitable breathing characteristics and determining, based on the one or more breathing characteristics, will be apparent to the skilled person. See, for example: Jiali Xie et al. (2021), "Audio-based snore detection using deep neural networks", Computer Methods and Programs in Biomedicine, 200:105917; Eliran Dafna et al. (2013), "Automatic detection of whole night snoring events using non-contact microphone", PloS One, 8(12):e84139; Erna Arnardottir et al. (2016), "How to measure snoring? A comparison of the microphone, cannula and piezoelectric sensor", Journal of Sleep Research, 25(2):158-168; D Sanchez Morillo et al. (2007), "Monitoring and analysis of cardio respiratory and snoring signals by using an accelerometer", Annual International Conference of the IEEE Engineering in Medicine and Biology Society, 2007:3942-5; Hyo-Ki Lee et al. (2013), "Automatic snoring detection from nasal pressure data", Annual International Conference of the IEEE Engineering in Medicine and Biology Society, 2013:6870-2; and Su Hwan Hwang et al. (2015), "Polyvinylidene fluoride sensor-based method for unconstrained snoring detection", Physiological Measurement, 36(7):1399-1414.

In response to a determination that the subject is snoring, the processing system 130 may control the pulse oximetry sensor 110 to start acquiring pulse oximetry data 115 (i.e. without further processing the one or more breathing characteristics), or process the one or more breathing characteristics to identify a type of snoring or to determine a running variance for a time interval between consecutive snores. The type of snoring or the running variance may be used to control the pulse oximetry sensor.

In other words, in some examples, the processing system 130 may turn on or activate the pulse oximetry sensor 110 as soon as it is determined that the subject 140 is snoring. If it is determined that the subject is not snoring, the processing system 130 may continue processing the one or more breathing characteristics to determine whether the subject is snoring.

If the subject 140 has not yet started snoring (i.e. if the processing system has not yet turned on/activated the pulse oximetry sensor 110 or has turned off/de-activated the pulse oximetry sensor 110 since a most recent snore), the processing system 130 may control the pulse oximetry sensor not to acquire pulse oximetry data.

References throughout the description to a subject's starting snoring and stopping snoring refer to the start and end respectively of a period of snoring, and not to the start and end of individual snores. A snoring period is characterized by the (relatively short) intervals between consecutive snores: a snore having an interval greater than a predetermined snore interval threshold from an immediately preceding snore is considered to belong to a different snoring period to the immediately preceding snore. Suitable thresholds for determining an end of a snoring period are described below.

In some examples, the processing system 130 controls the pulse oximetry sensor 110 based on a type of snoring, identified by processing the one or more breathing characteristics. The time (with respect to a period of snoring) at which an oxygen desaturation event is more likely to occur will depend on whether the snoring occurs before or after an obstructive apnea event or during a hypopnea event. The subject 140 may also snore during regular (i.e. non-obstructed) breathing, during which time an oxygen desaturation event is very unlikely to occur. By identifying which of these types of snoring corresponds to the subject's current snoring, the processing system 130 can control the pulse oximetry sensor 110 to acquire pulse oximetry data 115 only at times during which an oxygen desaturation event is likely to occur.

In some examples, the type of snoring may be identified based on reference data, comprising typical features of one or more breathing characteristics for each of a plurality of types of snoring. The plurality of types of snoring may comprise: a first type of snoring that occurs during hypopnea events; a second type of snoring that occurs before an obstructive apnea event (i.e. immediately preceding an obstructive apnea event); a third type of snoring that occurs after an obstruction is resolved (i.e. immediately following the end of obstructive apnea event); and/or a fourth type of snoring that occurs during regular (i.e. non-obstructed breathing).

In some examples, the type of snoring may be identified based on known breathing/snoring characteristics. For example, during an obstructive apnea event, the subject 140 will stop breathing, and no breathing sounds or vibrations/other movements associated with breathing will be detected. A period (e.g. of at least 10 seconds) during which no breathing is detected in the breathing data is therefore indicative of an obstructive apnea event. In particular, a bout of consecutive apnea events may be characterized by a pattern of snoring interspersed with brief periods (e.g. at least 10 seconds) of snoring/breathing cessation.

A resolution of an obstructive apnea or hypopnea event is often accompanied by a short period of snorting, gasping and/or other vocalizations that differ from regular (typically fixed frequency) snoring. Characteristics of such vocalizations (e.g. variations in frequency) may therefore be used to identify a type of snoring that occurs after an obstruction is resolved.

During hypopnea events, airflow is reduced, and snoring characteristics differ from the characteristics of regular snoring, and often change throughout the hypopnea event. For example, increases in the energy/envelope of the signal carrying the breathing data, corresponding to a progressive increase in respiratory effort, may be indicative of snoring that occurs during hypopnea events.

In some examples, the type of snoring may be identified using a machine-learning algorithm. A machine-learning algorithm is any self-training algorithm that processes input data in order to produce or predict output data. Here, the input data comprises one or more breathing characteristics (or simply the breathing data for periods during which snoring is detected) and the output data comprises an identification of a type of snoring.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example breathing characteristics of breathing data during periods of snoring (or example raw breathing data during periods of snoring). Preferably, the training input data entries are obtained from a large number of individuals. The training output data entries correspond to identifications of a type of snoring.

In some examples, the type of snoring may be identified based on subject-specific data obtained during at least one previous sleep session for the subject 140. The subject-specific data may comprise features of one or more breathing characteristics for the subject for each of a plurality of types of snoring. The plurality of types of snoring may comprise: a first type of snoring that the subject exhibits during hypopnea events; a second type of snoring that the subject exhibits before an obstructive apnea event (i.e. immediately preceding an obstructive apnea event); a third type of snoring that the subject exhibits after an obstruction is resolved (i.e. immediately following the end of obstructive apnea event); and/or a fourth type of snoring that the subject exhibits during regular (i.e. non-obstructed breathing).

The features of the one or more breathing characteristics for the subject for each of the plurality of types of snoring may be determined using breathing data responsive to a subject's breathing during at least one previous sleep session and data comprising times of detected obstructive apnea and hypopnea events during the same at least one previous sleep session. The data comprising times of detected obstructive apnea and hypopnea events may be obtained using any known technique for detecting such events, including a polysomnography (PSG), a home sleep apnea test (HSAT) or pulse oximetry.

The breathing data at one or more time periods (e.g. a period between 5 and 10 minutes long) during the at least one previous sleep session immediately preceding a detected obstructive apnea event (i.e. during one or more periods that each end at the start of a detected obstructive apnea event), at one or more time periods (e.g. a period between 5 and 10 minutes long) immediately following the end of a detected obstructive apnea event, at one or more time periods corresponding to a duration of a detected hypopnea event, and/or at one or more time periods corresponding to a duration of regular breathing may be processed to determine the features of the one or more breathing characteristics for each of the plurality of types of snoring.

In some examples, a machine-learning algorithm, trained using subject-specific data, may be used to identify the type of snoring. Suitable machine-learning algorithms for identifying a type of snoring that may be trained using subject-specific data will be apparent to the skilled person. Examples of suitable machine-learning algorithms include reinforcement learning algorithms, in particular, active reinforcement learning algorithms.

Active reinforcement learning may be used to maximize the detection of oxygen desaturation events while minimizing the time for which the pulse oximetry sensor is active. The pulse oximetry sensor may initially (i.e. before the machine learning algorithm is trained) run continuously throughout at least one sleep session (or continuously throughout a part of each sleep session, e.g. until the sensor's battery has run out of charge). The machine learning algorithm may then learn to detect types of snoring that occur before/after an obstructive apnea event and during a hypopnea event by being rewarded when the algorithm correctly identifies a type of snoring that indicates an oxygen desaturation event, and being punished when the algorithm identifies a type of snoring that indicates an oxygen desaturation event and no oxygen desaturation event occurs. In some examples, the pulse oximetry sensor may run continuously throughout each sleep session (or a part of each sleep session, e.g. e.g. until the sensor's battery has run out of charge) until sufficient examples of different types of snoring associated with apnea/hypopnea events have been found for the subject 140 (e.g. until a predetermined number of examples have been found for each type of snoring or until the machine-learning algorithm has achieved a predetermined level of accuracy).

Having identified a type of snoring, the processing system 130 may control the pulse oximetry sensor 110 according to which type of snoring has been identified.

For example, in response to a determination that the subject's current snoring corresponds to regular (i.e. non-obstructed) snoring, the processing system 130 may control the pulse oximetry sensor 110 not to acquire (or to continue not acquiring) pulse oximetry data. An oxygen desaturation event is very unlikely to occur during regular snoring, so this reduces a likelihood that the pulse oximetry sensor will acquire unnecessary data.

In response to a determination that the subject's current snoring corresponds to a type of snoring that occurs during a hypopnea event, the processing system 130 may control the pulse oximetry sensor 110 to start acquiring pulse oximetry data 115. In other words, the pulse oximetry sensor may be turned on/activated as soon as the processing system has determined that the subject 140 is snoring and that the snoring is a type that occurs during a hypopnea event. This allows pulse oximetry data to be acquired during hypopnea events throughout the sleep session of the subject without continuously acquiring pulse oximetry data.

In response to a determination that the subject's current snoring corresponds to a type of snoring that occurs before an obstructive apnea event, the processing system 130 may process the one or more breathing characteristics to determine a time since a most recent snore. The processing system may then control the pulse oximetry sensor 110 to start acquiring pulse oximetry data 115 in response to a determination that the time since a most recent snore exceeds a first predetermined time period. If the time since a most recent snore does not exceed the first predetermined time period, the processing system may continue to determine a time since a most recent snore until the time does exceed the first predetermined time period.

In other words, the pulse oximetry sensor 110 starts acquiring pulse oximetry data 115 as soon as it is determined that an obstructive apnea event is likely to have begun. A cessation of a type of snoring that occurs before an obstructive apnea event will generally indicate the start of an obstructive apnea event (during which the subject is not breathing, and therefore not snoring).

The first predetermined time period is a time period long enough to detect a cessation of snoring (i.e. long enough to distinguish a cessation of snoring from an interval between snores in a same period of snoring). The first predetermined time period may be less than a length of a typical breathing cycle (e.g. based on a population average or a subject-specific average breathing rate). For instance, the first predetermined time period may be half the length of a typical breathing cycle (e.g., for a breathing rate of 12 breaths per minute, the first predetermined time period may be 2.5 seconds). If the first predetermined time period is based on a subject-specific average breathing rate, the processing system may process the breathing data 125 to determine a breathing rate for the subject during regular breathing, and determine the first predetermined time period based on the determined breathing rate.

In response to a determination that the subject's current snoring corresponds to a type of snoring that occurs after an obstruction is resolved, the processing system 130 may control the pulse oximetry sensor 110 to start acquiring pulse oximetry data 115. In other words, the pulse oximetry sensor may be turned on/activated as soon as the processing system determines that an obstructive apnea event has ended. Acquiring pulse oximetry data immediately after the end of an obstructive apnea event should result in the detection of oxygen desaturation, as it will take a few breathing cycles (e.g. up to a minute) for the subject's oxygen saturation level to return to normal (i.e. to the oxygen saturation level before any obstructive apnea and/or hypopnea events had occurred).

In some examples, the processing system 130 controls the pulse oximetry sensor 110 based on one or more inter-snore variability characteristics. The one or more inter-snore variability characteristics are characteristics representative of an inter-snore variability, and may include any suitable statistical parameters (e.g. variance, standard deviation interquartile range etc.). For instance, the one or more inter-snore variability characteristics may include a running variance for one or more snore features, where the one or more snore features includes at least one of a snore energy, a snore duration, a time interval between consecutive snores, a fundamental frequency of the signal during snoring, an amplitude of the signal envelope, and/or a change (e.g. a slope or a range) in any of these characteristics.

The processing system 130 may, in response to a determination that the subject 140 is snoring, process the one or more breathing characteristics to determine a running variance for each of the one or more snore features. The running variance(s) may be determined each time a new snore is detected in the breathing data. The processing system may then control the pulse oximetry sensor to acquire pulse oximetry data 115 in response to a determination that each running variance exceeds a predetermined running variance threshold, and not to acquire pulse oximetry data in response to a determination that the running variance for one or more snore features does not exceed the predetermined threshold. For example, the predetermined energy running variance threshold may be a value between 0.2 and 0.3. Preferably the predetermined running variance threshold has a value in the range 0.24-0.26 (e.g. 0.25).

The running variance for each of the one or more snore features may be determined as a within-group running variance for the one or more snore features, where a group of snores is a plurality of snores for which each time interval between consecutive snores is below a predetermined time interval threshold (e.g. 60 seconds). If a snore has a time interval from an immediately preceding snore that is greater than the time interval threshold, the snore is considered to belong to a different group to the preceding snore. In other words, the running variance for each of the one or more features may be determined based only on features of snores in a current group of snores (i.e. the group of snores to which a most recent snore belongs). If the time interval between the most recent snore and an immediately preceding snore exceeds the predetermined time interval threshold, the most recent snore is the first snore in the current group of snores.

As described in Nir Ben-Israel et al. (2012), "Obstructive apnea hypopnea index estimation by analysis of nocturnal snoring signals in adults", SLEEP, 35(9): 1299-13 05 C, the running variance is found to be higher adjacent to apnea events than during simple snoring phases (i.e. snoring associated with regular breathing). Controlling the pulse oximetry sensor based on running variance therefore effectively classifies the subject's current snoring as either regular snoring or apneic snoring and controls the pulse oximetry sensor to acquire pulse oximetry data only during apneic snoring.

In some examples, the one or more inter-snore variability characteristics may be used in combination with other characteristics to control the pulse oximetry sensor 110. Characteristics used in combination with the one or more inter-snore variability characteristics to determine when to control the pulse oximetry sensor to start and/or stop controlling pulse oximetry data may include time relations between groups of snoring events.

In some examples, the one or more breathing characteristics may be used to determine when to stop acquiring pulse oximetry data (i.e. when to turn off/deactivate the pulse oximetry sensor). For instance, the processing system 130 may process the one or more breathing characteristics to determine a time since a most recent snore. The processing system may then control the pulse oximetry sensor 110 to stop acquiring pulse oximetry data 115 in response to a determination that the time since a most recent snore exceeds a second predetermined time period. If the time since a most recent snore does not exceed the second predetermined time period, the processing system may control the pulse oximetry sensor to continue acquiring pulse oximetry data until the time since a most recent snore exceeds the second predetermined time period.

In this way, the pulse oximetry sensor 110 continues to acquire pulse oximetry data 115 for the period during which oxygen desaturation is likely to occur, and conserves battery power by stopping acquiring pulse oximetry data once oxygen desaturation is no longer likely to occur.

The second predetermined time period may be long enough, not only to detect a cessation of snoring, but to ensure that pulse oximetry data is acquired in a period immediately after the cessation of snoring. The cessation of snoring may be caused by the start of an obstructive apnea event, in which case the subject's oxygen saturation level will not begin to fall until after the subject has stopped snoring. For instance, a second predetermined time period of 15-30 seconds from a most recent snore may be long enough to acquire useful pulse oximetry data while reducing the amount of unnecessary pulse oximetry data collected. Preferably, the second predetermined time period has a value between 20 and 25 seconds.

Other mechanisms for determining when to stop acquiring pulse oximetry data based on the one or more breathing characteristics will be apparent to the skilled person. In particular, in examples in which a type of snoring is identified, a time after which oxygen saturation is no longer likely can be determined more precisely. For instance, if the subject's snoring is a type of snoring that occurs after an obstructive apnea event, the subject's oxygen saturation level may have returned to normal before the subject has stopped snoring. In such a case, the processing system may instead control the pulse oximetry data to stop acquiring pulse oximetry data once a time since the subject started the period of snoring has exceeded a predetermined time period.

In some examples, obtained pulse oximetry data may additionally or alternatively be used to determine when to stop acquiring pulse oximetry data. The processing system 130 may obtain pulse oximetry data 115 from the pulse oximetry sensor 110, and process the pulse oximetry data to determine the measure of oxygen saturation. The processing system may then control the pulse oximetry sensor based on the determined measure of oxygen saturation.

For instance, the processing system 130 may control the pulse oximetry sensor 110 to stop acquiring pulse oximetry data 115 in response to a determination that the measure of oxygen saturation exceeds an oxygen saturation threshold throughout a third predetermined time period. In other words, the pulse oximetry sensor may be turned off/deactivated if the measure of oxygen saturation does not fall below the oxygen saturation threshold during the third predetermined time period (i.e. if no oxygen desaturation events are detected).

The third predetermined time period may start at a time at which the pulse oximetry sensor 110 most recently started acquiring pulse oximetry data 115 (i.e. when the pulse oximetry sensor was last turned on/activated). The third predetermined time period may be long enough that an oxygen desaturation event would have been expected to occur before the end of the third predetermined time period had an obstructive apnea or hypopnea event occurred. The length of the third predetermined time period may therefore depend on which mechanism used to start acquiring pulse oximetry data, and, where a type of snoring is used to control the pulse oximeter sensor, the type of snoring identified.

For example, if the pulse oximetry sensor 110 starts acquiring pulse oximetry data 115 in response to the cessation of a type of snoring that occurs before an obstructive apnea event, an obstructive apnea event would be expected to have occurred shortly before the pulse oximetry sensor started acquiring the pulse oximetry data. A third predetermined time period of a few seconds (preferably less than 15 seconds, e.g. 10 seconds) would therefore be sufficient: if an obstructive apnea event had occurred, it is highly likely that oxygen desaturation would have been detected by the end of this period.

The oxygen saturation threshold, used to determine whether or not an oxygen desaturation event has occurred, may be a predetermined threshold, e.g. based on typical clinical thresholds for detecting oxygen desaturation events. For example, the oxygen saturation threshold may have a value of 95% (as an oxygen saturation level above 95% is typically considered normal).

Alternatively, the oxygen saturation threshold may be a subject-specific threshold, based, for example, on one or more values of the measure of oxygen saturation obtained during a period of regular breathing, or on a value of the measure of oxygen saturation determined from pulse oximetry data acquired at the start of a current pulse oximetry data acquisition period (where a current acquisition period is a period since the pulse oximetry sensor most recently started acquiring pulse oximetry data).

For example, the oxygen saturation threshold may be a value corresponding to a 3% fall in oxygen saturation from a pre-event baseline value (e.g. an oxygen saturation value from the period immediately preceding a decline in oxygen saturation). A 3% fall in oxygen saturation is one of the criteria used by the American Academy of Sleep Medicine (AASM) to score hypopnea events (see, for example, version 2.5: Berry RB, Albertario CL, Harding SM, et al., The AASM Manual for the Scoring of Sleep and Associated Events. 2.5. American Academy of Sleep Medicine; 2018). In some examples, the processing system 130 may control the pulse oximetry sensor 110 to start acquiring pulse oximetry data 115 in response to a determination that all of the AASM's criteria for scoring hypopnea events have been met (i.e. that the oxygen saturation has fallen by at least 3% from a pre-event baseline value, that the peak signal excursions have dropped by at least 30% from a pre-event baseline, and that the duration of the drop in peak signal excursion is at least 10 seconds).

In some examples, the processing system 130 may control the pulse oximetry sensor 110 to stop acquiring pulse oximetry data 115 in response to a determination that the measure of oxygen saturation had fallen below the oxygen saturation threshold since the pulse oximetry sensor most recently started acquiring pulse oximetry data, and that the measure of oxygen saturation now exceeds the oxygen saturation threshold. In other words, the pulse oximetry sensor may be controlled to stop acquiring pulse oximetry data in response to a determination that an oxygen desaturation event has ended.

Figure 2 illustrates a computer-implemented method 200 for controlling a pulse oximetry sensor, according to an embodiment of the invention.

The method begins at step 210, at which breathing data responsive to a subject's breathing is obtained, during a sleep session of the subject, from a detector different to the pulse oximetry sensor.

At step 220, the breathing data is processed to determine one or more breathing characteristics.

At step 230, the pulse oximetry sensor is controlled responsive to the one or more breathing characteristics.

It will be understood that the disclosed methods are computer-implemented methods. As such, there is also proposed a concept of a computer program comprising code means for implementing any described method when said program is run on a processing system.

The skilled person would be readily capable of developing a processor for carrying out any herein described method. Thus, each step of a flow chart may represent a different action performed by a processor, and may be performed by a respective module of the processor.

As discussed above, the system makes use of a processor to perform the data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A processing system for controlling a pulse oximetry sensor, the processing system being configured to:
obtain, from a detector different to the pulse oximetry sensor, breathing data responsive to a subject's breathing during a sleep session of the subject;
process the breathing data to determine one or more breathing characteristics; and
control the pulse oximetry sensor responsive to the one or more breathing characteristics.

2. The processing system of claim 1, wherein the detector comprises at least one of: a microphone, a resonator, an accelerometer, a gyroscope, an inertial measurement unit, a pressure sensor, a flow sensor, a seismocardiographic sensor, and/or a ballistocardiographic sensor.

3. The processing system of claim 1 or 2, wherein the processing system is configured to control the pulse oximetry sensor by:
processing the one or more breathing characteristics to determine whether the subject is snoring; and
controlling the pulse oximetry sensor to start acquiring pulse oximetry data in response to a determination that the subject is snoring.

4. The processing system of claim 1 or 2, wherein the processing system is configured to control the pulse oximetry sensor by:
processing the one or more breathing characteristics to determine whether the subject is snoring; and, in response to a determination that the subject is snoring:
processing the one or more breathing characteristics to identify a type of snoring; and
controlling the pulse oximetry sensor according to the identified type of snoring.

5. The processing system of claim 4, wherein the processing system is configured to, in response to the identified type of snoring being a first type of snoring that occurs during hypopnea:
control the pulse oximetry sensor to start acquiring pulse oximetry data.

6. The processing system of claim 4 or 5, wherein the processing system is configured to, in response to the identified type of snoring being a second type of snoring that occurs before an obstructive apnea event:
process the one or more breathing characteristics to determine a time since a most recent snore; and
control the pulse oximetry sensor to start acquiring pulse oximetry data in response to a determination that the time since a most recent snore exceeds a first predetermined time period.

7. The processing system of any of claims 4 to 6, wherein the processing system is configured to, in response to the identified type of snoring being a third type of snoring that occurs after an obstruction is resolved:
control the pulse oximetry sensor to start acquiring pulse oximetry data.

8. The processing system of any of claims 4 to 7, wherein the processing system is configured to identity a type of snoring based on subject-specific data obtained during at least one previous sleep session for the subject.

9. The processing system of claim 8, wherein the processing system is configured to identify a type of snoring using a machine-learning algorithm, wherein the machine-learning algorithm has been trained using the subject-specific data obtained during at least one previous sleep session for the subject.

10. The processing system of claim 1 or 2, wherein the processing system is configured to control the pulse oximetry sensor by:
processing the one or more breathing characteristics to determine whether the subject is snoring; and, in response to a determination that the subject is snoring:
processing the one or more breathing characteristics to determine a running variance for one or more snore features;
in response to a determination that each running variance exceeds a predetermined running variance threshold, controlling the pulse oximetry sensor to acquire pulse oximetry data; and
in response to a determination that the running variance for one or more snore features does not exceed the predetermined threshold, controlling the pulse oximetry sensor not to acquire pulse oximetry data.

11. The processing system of any of claims 1 to 10, wherein the processing system is configured to control the pulse oximetry sensor by:
processing the one or more breathing characteristics to determine a time since a most recent snore; and
controlling the pulse oximetry sensor to stop acquiring pulse oximetry data in response to a determination that the time since a most recent snore exceeds a second predetermined time period.

12. The processing system of any of claims 1 to 11, wherein the processing system is configured to control the pulse oximetry sensor by:
obtaining pulse oximetry data from the pulse oximetry sensor;
processing the pulse oximetry data to determine a measure of oxygen saturation; and
controlling the pulse oximetry sensor to stop acquiring pulse oximetry data in response to a determination that the measure of oxygen saturation exceeds an oxygen saturation threshold throughout a third predetermined time period.

13. A system for measuring oxygen saturation, comprising:
a pulse oximetry sensor;
a detector capable of obtaining breathing data responsive to a subject's breathing during a sleep session of the subject; and
the processing system of any of claims 1 to 12.

14. A computer-implemented method for controlling a pulse oximetry sensor, the computer-implemented method comprising:
obtaining, from a detector different to the pulse oximetry sensor, breathing data responsive to a subject's breathing during a sleep session of the subject;
processing the breathing data to determine one or more breathing characteristics; and
controlling the pulse oximetry sensor responsive to the one or more breathing characteristics.

15. A computer program product comprising computer program code means which, when executed on a computer device having a processing system, cause the processing system to perform all of the steps of the method according to claim 14.
